# EUROPEAN PATENT APPLICATION

(11) **EP 0 584 489 A1**
(43) Date of publication of application: **02.03.1994**
(21) Application number: 93110251.1
(22) Date of filing: 28.06.1993
(51) Int. Cl.: A61B 5/103, G01B 3/30, A61B 17/11

(54) **Sizing device for sizing an opening in a hollow body organ**

(30) Priority: 25.08.1992 US 934320
(71) Applicant: AMERICAN CYANAMID COMPANY, Stamford Connecticut 06904-0060 (US)
(72) Inventor: Vitali, Dario, Monroe, Connecticut 06468 (US); Keller, John W., Huntington, Connecticut 06484 (US); Guinn, Clayton P., Woodstock, Georgia 30188 (US); Michalski, Rhonda T., Bayside, New York 11364 (US)
(74) Representative: Wächtershäuser, Günter, Prof. Dr.

(57) **Abstract**

A sizing device includes a handle (16), a neck (18) joined at its proximal end to the handle (16), and a sizing head (12) connected to a distal end of the neck (18). The sizing head (12) has a continuously uninterrupted surface so as to be easily inserted into an opening in a hollow body organ to measure the diameter of the opening. The head (12) can be comprised of a plurality of cylindrical sizing rings (22-25) of different predetermined diameters increasing in size from a distal end of the head. Alternatively, the sizing head (12) can be conically shaped with a stepless outer surface.

## Description

This invention relates generally to a surgical device, and more particularly to a sizing device for measuring the lumen diameter of a tubular body organ or the circumference of any other body organ opening.

For example, after a surgical procedure such as cutting and removing a diseased or cancerous portion of a hollow body organ such as the bowel the severed ends of the bowel must be anastomosed or reconnected. The subject invention is particularly useful for sizing the lumen diameter of the bowel sections prior to the anastomosis procedure.

One surgical procedure for anastomosing a hollow body organ uses an particular two-piece anastomotic device known in the prior art. The anastomotic device comprises a pair of ring members to be placed in the open ends of a severed body organ to be anastomosed. The ring members have annular connecting means which mate with each other to clamp the open ends contiguous to each other so they can grow and heal together.

Again, for example, when anastomosing the bowel sections of a patient, one of the ring members, i.e., the distal ring member, is usually inserted into the open end of the upper bowel. The other ring member, i.e., the proximal ring member, may then be inserted into the open end of the lower bowel. The ring members can be secured to the respective open ends of the bowel by purse string sutures and are then closed together.

To avoid, as much as possible, patient trauma, or even worse, serosal tearing of the bowel, a properly sized anastomotic ring member should be used. Specifically, the ring member should have a maximum diameter which comfortably fits the smallest lumen diameter of the bowel segments being rejoined. The sizing device of the subject invention may be used to measure quickly and accurately the lumen diameter of the two bowel segments to be rejoined to ensure that a properly sized ring member is used.

Of course, the device of the present invention can be used with equal advantage in surgical anastomosis procedures other than resection of the bowel.

Conventional means for measuring the lumen diameter of the lower bowel include individually sized measuring devices each of which includes an elongate handle that carries an egg-shaped head. The devices are assembled in a set with each device in the set having a head of different maximum diameter. The diameter of the opening in the organ, such as the bowel segments, is determined by selecting devices from the set and inserting them into the opening until one that produces a close fit is found. However, this procedure of inserting individually sized measuring devices into the organ opening is time consuming and usually requires the use of several differently sized devices on a trial by error basis.

One prior art sizing gauge includes a plurality of cylindrical sizing elements on one or both ends of a handle. The sizing elements are of different known diameters and arranged in a stepped configuration starting with the smallest diameter sizing element. However, the sizing gauge is designed for measuring the interior diameter of a vascular vessel to which a graft is to be surgically attached. Therefore, the stepped cylindrical sizing elements are each shaped to have a circumferential bevelled portion and a circumferential flat shoulder portion about their distal end. When the sizing gauge is inserted into the open end of the vascular vessel, proper sizing is achieved when a sizing element fits snugly in the vessel and the open end abuts the flat shoulder portion of the next largest sizing element.

When replacing a defective vascular section with a tubular graft element, it is important that the lumen diameter of the graft precisely matches that of the vessel to which it will be surgically attached. Therefore, toward that end, the Hodge device uses the stepped cylindrical sizing elements with flat shoulder portions. The flat shoulder portion approximates the open end of the tubular graft and thus provides an accurate measurement for sizing the vascular vessel and choosing a suitably sized tubular graft.

However, providing sizing elements with flat shoulder portions is not always necessary, and can even have drawbacks. Significantly, since the flat shoulder portion provides a surface which is substantially perpendicular to the inner surface of the hollow body organ, it can reduce the continuity of the contour surface of the sizing device and make it more difficult to be inserted into an extended vascular section. However, the sizing device should fit as smoothly as possible into the tubular organ.

As further improvements in devices for measuring the lumen diameter of a tubular body organ are needed, the present invention provides an economical, effective, and easy-to-use sizing device.

For purposes of this specification and the concluding claims, it will be understood that the term "sizing" as used hereinafter is intended to encompass sizing of the open end of a tubular organ or other opening in any other body organ. Thus, as used in the specification and concluding claims, the term "tubular body organ" is intended to encompass not only organs like the bowel but also generally others, such as the stomach, that might not be considered to be "tubular" in the strict sense of the word.

It is a principal object of the present invention to provide an easy-to-use sizing device for measuring the lumen diameter of a tubular body organ.

Accordingly, it is an object of the invention to provide a sizing apparatus that quickly and accurately measures the lumen diameter of a tubular body organ or other opening in a body organ.

It is a further object of the invention to provide a sizing apparatus with a streamlined contour surface for ease of insertion into a hollow body organ.

In accordance with one aspect of the invention, the sizing device comprises a handle, a neck joined at its proximal end to the handle, and a sizing head connected to a distal end of the neck. The sizing head includes a plurality of cylindrical sizing rings of different predetermined diameters, with the sizing rings increasing in diameter from a distal end of the head. Each sizing ring has a circumferential bevelled portion at its distal end to provide a continuous uninterrupted surface to the next smallest sizing ring.

In accordance with another aspect of the invention, a sizing device comprises a handle, a neck joined at its proximal end to the handle, and a sizing head connected to a distal end of the neck. The sizing head is conically shaped and has a stepless outer surface, with the head continuously increasing in diameter from its distal end.

In accordance with yet another aspect of the present invention, a sizing device comprises a handle, a neck having a distal end and a proximal end and joined at the proximal end to the handle, and a sizing head having a distal end and a proximal end, and being connected at its proximal end to the distal end of the neck. The sizing head has a plurality of cylindrical sizing rings of different known diameters, with the sizing rings increasing in diameter from the distal end of the sizing head and having a lead sizing ring of the smallest diameter positioned at the distal end of the sizing head and contiguous with at least one secondary sizing ring. Each secondary sizing ring has a circumferential rounded surface at its distal end to provide a continuous uninterrupted surface to the next smallest sizing ring.

These and other objects, aspects, features and advantages of the present invention will become apparent from the following detailed description of the preferred embodiments taken in conjunction with the accompanying drawings.
Figure 1 is a perspective view of a first embodiment of the sizing device in accordance with the present invention;
Figure 2 is a side elevational view of the sizing device with an alternatively shaped handle in accordance with the present invention;
Figure 3 is a side elevation view of the sizing device with an alternatively shaped handle in accordance with the present invention;
Figure 4 is a side elevational view of a sizing head in accordance with the first embodiment of the present invention;
Figure 5 is a side elevational view of a sizing head in accordance with a second embodiment of the present invention;
Figure 6 is a side elevational view of the sizing device in accordance with a third embodiment of the present invention;
Figure 7 is a side elevational view of a sizing head in accordance with another embodiment of the present invention;
Figure 8 is a front elevational view of the sizing head shown in Figure 7 in accordance with the present invention;
Figure 9 is a side elevational view of still another embodiment, with the head partially broken away to show internal detail;
Figure 10 is a rear elevational view of the embodiment of Figure 9; and
Figure 11 is a front elevational view of the embodiment of Figures 9 and 10.

For ease of reference, as used herein the term "distal" will refer to that part of the device which is farthest from the surgeon-user, and the term "proximal" refers to that part of the device which is closest to the surgeon-user.

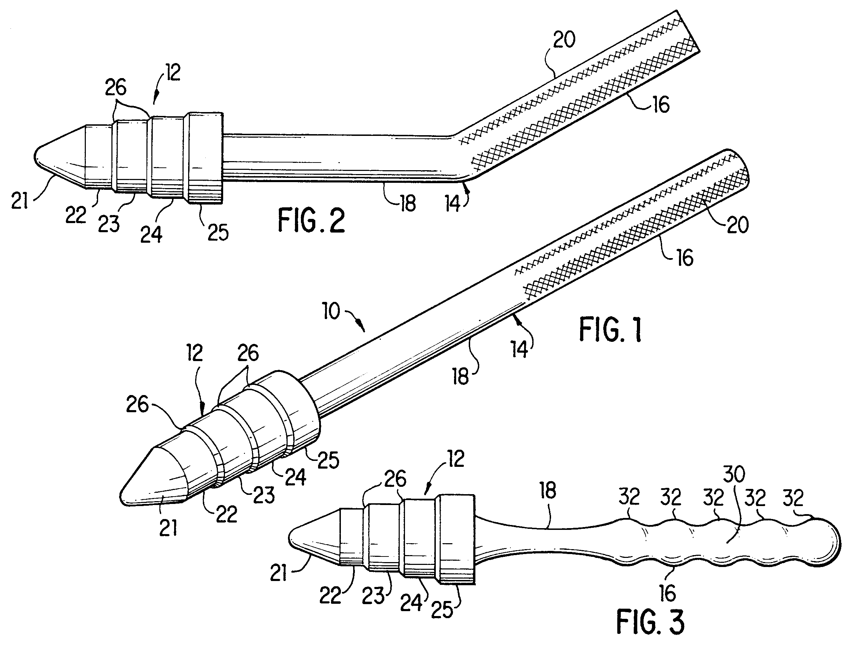

The sizing device or sizer 10, which may be used for sizing organs in the gastrointestinal tract or openings in other hollow body organs, in accordance with a first embodiment of the present invention is shown in Figures 1 through 4. The sizer 10 comprises a sizing head 12 and a shaft member 14, with the shaft member having a handle 16 and a neck 18.

Shaft member 14 in Figure 1 is a cylindrically shaped straight piece of material made of, for example, stainless steel. The handle portion is preferably provided with a knurled surface 20 to aid in gripping.

Sizing head 12 is connected to a distal end of the neck and is comprised of a plurality of cylindrical sizing rings 22 through 25. The sizing rings are made from a surgical grade material such as stainless steel. Although four sizing rings are shown in Figure 1, this number is arbitrary and can vary without departing from the scope of the invention. The sizing rings increase in diameter in small increments from the forward-most ring 22 to the largest ring 25 at the proximal end of the sizing head. The smallest ring 22 includes a conical nose portion 21 for entry into the opening in the body organ. Circumferential bevelled portions 26 formed on the distal end of each sizing ring join adjacent sizing rings and provide the sizing head with a continuous uninterrupted contoured surface. This streamlined contoured surface enables the sizing head to gently slide into the opening in the body organ, such as the bowel, with relative ease until proper sizing is achieved.

Figures 2 and 3 show alternatively shape shafts for the gastrointestinal tract or other hollow body organ opening sizing device.

In Figure 2 the handle 16 is angled from neck 14 by, for example, approximately 30°. Depending upon the preference of the surgeon-user, the angled shaft can make the sizing device easier to manipulate. The handle in Figure 2 also has a knurled surface 20 like the handle shown in Figure 1. The handle in Figure 3 is provided with a contoured gripping surface 30 having a plurality of bulbous portions 32 for comfortably fitting between the fingers or in the palm of the surgeon-user's hand.

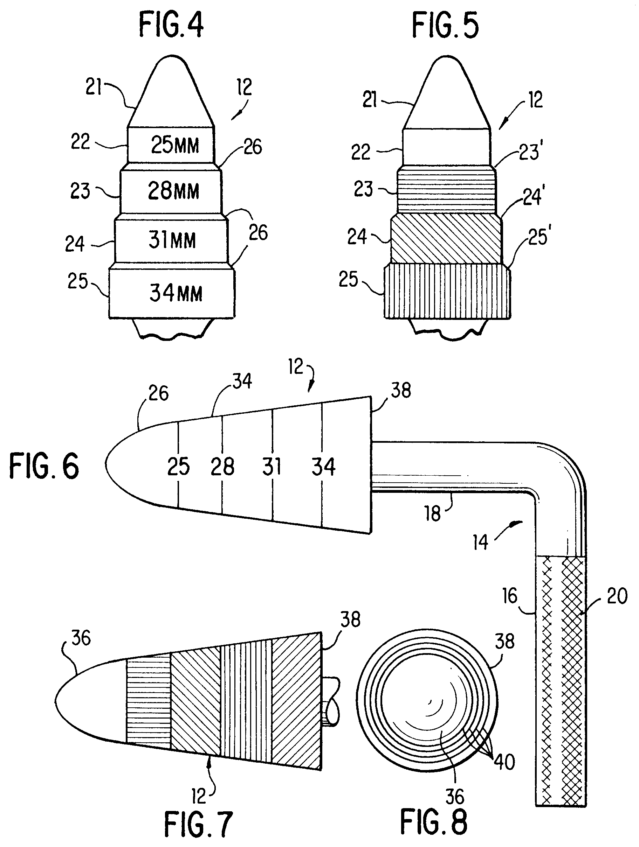

As shown in Figure 4, the sizing head of the first embodiment includes numerical indicia on each sizing ring to allow for easy visual determination of the proper size of the measured hollow body organ.

Figure 5 shows a second embodiment of the present invention. The sizing head 12 of the second embodiment differs structurally from the sizing device of the first embodiment by replacing the bevelled portions with rounded surfaces 23' through 25' on secondary sizing rings 23 through 25. The lead sizing ring 22, which is positioned at the distal end of the sizing head and is thus the smallest in diameter, still has the conical nose portion 21 as described in the first embodiment. The rounded surfaces provide the sizing head with substantially the same continuous uninterrupted contour surface as the bevelled portions but further reduce the costs of manufacturing the sizing rings. The sizing head of the second embodiment can be connected to, for example, any of the shafts shown in Figure 1 through 3.

As an alternative to the numerical indicia shown in the first embodiment, the second embodiment of Figure 5 illustrates color-coded sizing rings. As an example, sizing ring 25 could be colored red, sizing ring 24 colored green, sizing ring 23 colored blue and sizing ring 22 colored white.

A third embodiment of the present invention is shown in Figures 6 through 8. This embodiment differs from the first two embodiments by having a head 34 which continuously increases in diameter in a stepless manner to provide an even more streamlined contoured surface. As shown in Figures 6 and 7, the entire sizing head is conically shaped with a rounded nose portion 36 at its distal end and the largest diameter being at the base 38, or proximal end. The head also includes either numerical indicia as shown in Figure 6 or color-coded indicia as shown in Figure 7 for indicating the diameter of the head.

The handle 16 in Figure 6 is angled at approximately 90° to the neck 18 and includes a knurled surface 20.

The front view of the sizing head in Figure 8 shows rounded nose portion 36 and a plurality of concentric rings 40. The rings delineate the different color sections on the sizing head 12 shown in Figure 7.

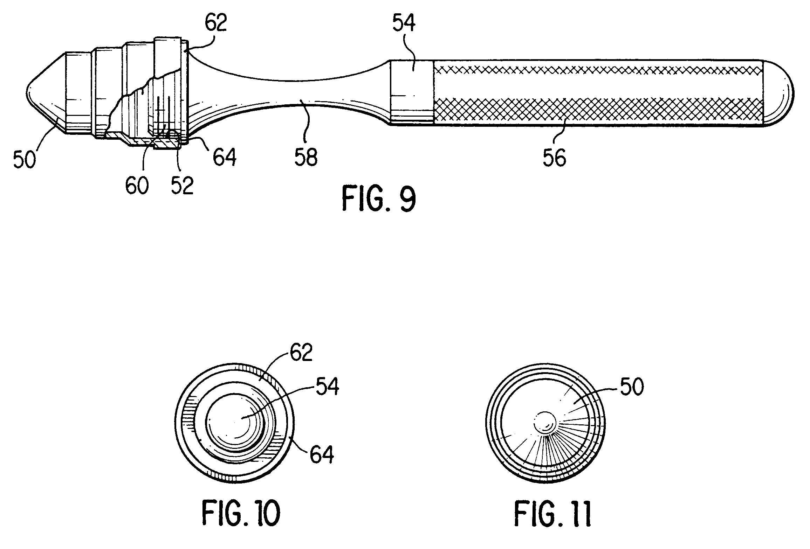

Figures 9 to 11 show yet another embodiment of the present invention. This embodiment includes a head 50 having an exterior shape substantially the same as that shown in Figures 1 through 5. However, the head 50 may have an exterior shape such as that shown in Figures 6 through 8. As can be seen in Figure 9, the proximal end of the head 50 is internally threaded at 52. A handle 54 has a knurled proximal end 56 and a reduced diameter neck 58 that terminates in an externally threaded distal end 60 bounded by a flange 62. The threaded handle end 60 is mated with the threaded head end 52 to assemble the device and the flange 62 abuts the proximal end surface 64 of the head to act as a stop for the threaded engagement.

To use the sizing device, the head is inserted into the opening in a hollow body organ, such as a bowel section of the intestinal tract. The sizing device is inserted further in the opening by manipulating the shaft member until it can no longer be gently moved forwardly. The largest diameter sizing ring that comfortably fits into, for example, the bowel section, indicates its diameter.

In this manner, a ring member of the *VALTRAC*® device can be chosen according to the measured size of the diameter of the opening in the body organ and can be placed in the opening thereafter to be provided with a purse string suture.

Although specific embodiments of the present invention have been described above in detail, it will be understood that this description is merely for purposes of illustration. Various modifications of and equivalent structures corresponding to the disclosed aspects of the preferred embodiments in addition to those described above may be made by those skilled in the art without departing from the spirit of the present invention which is defined in the following claims, the scope of which is to be accorded the broadest interpretation so as to encompass such modifications and equivalent structures.

## Claims

1. A sizing device, comprising:
a handle;
a neck joined at its proximal end to said handle; and
a sizing head connected to a distal end of said neck and including a plurality of cylindrical sizing rings of different predetermined diameters, said sizing rings increasing in diameter from a distal end of said sizing head, and each sizing ring having a circumferential bevelled portion at its distal end to provide a continuous uninterrupted surface to the next smallest sizing ring.

2. A sizing device according to Claim 1, further comprising a conically-shaped nose portion contiguous with a distal end of said sizing ring having the smallest diameter.

3. A sizing device according to Claim 1, wherein said handle and said neck extend along a common longitudinal axis.

4. A sizing device according to Claim 1, wherein said handle extends at an angle from said neck.

5. A sizing device according to Claim 1, further comprising numerical indicia on each said sizing ring to indicate its diameter.

6. A sizing device according to Claim 1, wherein each said sizing ring is a different color to indicate its diameter.

7. A sizing device, comprising:
a handle;
a neck joined at its proximal end to said handle; and
a sizing head connected to a distal end of said neck, said head being conically shaped and having a stepless outer surface, with said sizing head continuously increasing in diameter from its distal end.

8. A sizing device according to Claim 7, wherein said distal end of said sizing head comprises a rounded nose portion.

9. A sizing device according to Claim 7, wherein said sizing head includes numerical indicia to delineate different diameters.

10. A sizing device according to Claim 7, wherein said sizing head is divided into a plurality of circumferential bands having different colors to delineate different diameters.

11. A sizing device according to Claim 7, wherein said handle extends at an angle from said neck.
